Europäisches Patentamt

European Patent Office

Office européen des brevets

(11) Veröffentlichungsnummer: **0 026 847**
**B1**

(12) **EUROPÄISCHE PATENTSCHRIFT**

(45) Veröffentlichungstag der Patentschrift:
23.06.82

(51) Int. Cl.³: **C 07 D 237/22**

(21) Anmeldenummer: 80105271.3

(22) Anmeldetag: 04.09.80

(54) Verfahren zur Herstellung von 4-Amino-5-chlor-1-phenyl-pyridazon-(6).

(30) Priorität: 15.09.79 DE 2937421

(43) Veröffentlichungstag der Anmeldung:
15.04.81 Patentblatt 81/15

(45) Bekanntmachung des Hinweises auf die Patenterteilung:
23.06.82 Patentblatt 82/25

(84) Benannte Vertragsstaaten:
AT BE CH DE FR GB IT LI NL SE

(56) Entgegenhaltungen:
**DD-A-116 615**
**DD-A-124 918**
**DE-A-1 620 186**
**DE-A-2 925 110**
**GB-A-871 674**

(73) Patentinhaber: **BASF Aktiengesellschaft,
Carl-Bosch-Strasse 38, D-6700 Ludwigshafen (DE)**

(72) Erfinder: **Richarz, Winfried, Dr., Königsberger Strasse 5,
D-6081 Stockstadt/Rh. (DE)**
Erfinder: **Froehlich, Helmut, Dr., Dirmsteiner Weg 35,
D-6700 Ludwigshafen (DE)**
Erfinder: **Schroeder, Harald, Dr., Lisztstrasse 168,
D-6700 Ludwigshafen (DE)**

Verfahren zur Herstellung von 4-Amino-5-chlor-1-phenyl-pyridazon-(6)

Die vorliegende Erfindung betrifft ein Verfahren zur Herstellung von weitgehend reinem 4-Amino-5-chlor-1-phenyl-pyridazon-(6) durch Umsetzung von 4,5-Dichlor-1-phenyl-pyridazon-(6) mit Ammoniak in Gegenwart von Phenol-4-sulfonsäure.

Es ist bekannt, 4-Amino-5-chlor-phenyl-pyridazon-(6) als Herbizid zu verwenden, insbesondere zur selektiven Bekämpfung von Unkräutern in Zuckerrüben. Die Herstellung erfolgt nach der britischen Patentschrift 871 674 durch Umsetzung von 4,5-Dichlor-1-phenyl-pyridazon-(6) mit wäßrigem Ammoniak unter Druck und erhöhten Temperaturen. Dabei erhält man ein Gemisch von ca. 80% (Gew.-%) 4-Amino-5-chlor-1-phenylpyridazon-(6) und etwa 20% der isomeren 5-Amino-4-chlor-1-phenyl-pyridazon-(6)-verbindung, die ein weniger gutes Herbizid ist. Um Pflanzenschutzmittel mit möglichst hohem Gehalt an biologisch aktivem Wirkstoff herzustellen, benötigt man technische Wirkstoffe mit maximal erreichbarer Reinheit. Aus der DE-OS 1 620 186 ist es bekannt, die unerwünschte isomere Verbindung aus der bei der Herstellung erhaltenen Mischung durch Extraktion mit unpolaren Lösungsmitteln aus der Gruppe der aromatischen und hydroaromatischen Kohlenwasserstoffe weitgehend zu entfernen.

Es ist ferner bekannt, die Umsetzung des 4,5-Dichlorphenylpyridazons mit Ammoniak in organischen Lösungsmitteln durchzuführen, wobei ein Produkt hoher Reinheit erhalten wird (DD-PS 131 172). Die Verwendung organischer Lösungsmittel ist jedoch nachteilig.

Es wurde nun gefunden, daß man 4-Amino-5-chlor-1-phenyl-pyridazon-(6) leicht in ausgezeichneten Ausbeuten und hoher Reinheit erhält, wenn man 4,5-Dichlor-1-phenyl-pyridazon-(6) mit einer wäßrigen Lösung von Ammoniak bei einer Temperatur von 20 bis 200° und einem Druck von 1 bis 20 bar in Gegenwart von Phenol-4-sulfonsäure umsetzt.

Das erfindungsgemäße Verfahren hat zwei wesentliche Vorteile:

Erstens ist durch die Verwendung von Wasser als Reaktionsmedium das Verfahren überaus einfach und damit wirtschaftlich, da die Abtrennung des wasserunlöslichen Endproduktes durch einfaches Absaugen des Reaktionsgemisches möglich ist und dabei gleichzeitig das gut wasserlösliche Ammoniumsalz der Phenol-4-sulfonsäure vom Endprodukt abgetrennt wird. Zweitens beeinflußt die Phenol-4-sulfonsäure das Isomerenverhältnis bei der Umsetzung direkt, während bei den bekannten Verfahren die unerwünschte isomere Verbindung zwar entsteht aber durch Verwendung organischer Lösungsmittel abgetrennt wird.

Im Vergleich zu diesen Verfahren liefert das erfindungsgemäße Verfahren auf wesentlich einfacherem und wirtschaftlicherem Wege das gewünschte 4-Aminopyridazonderivat in ca.

90%iger Ausbeute und 95- bis 99%iger Reinheit.

Die Umsetzung kann beispielsweise folgendermaßen durchgeführt werden: In einem Druckbehälter werden 4,5-Dichlor-1-phenyl-pyridazon-(6), wäßrige Ammoniaklösung und Phenol-4-sulfonsäure vorgelegt, der Behälter wird geschlossen, die Mischung wird auf die gewünschte Reaktionstemperatur erwärmt und mehrere Stunden bei dieser Temperatur gehalten. Vorteilhaft werden die Reaktionskomponenten dabei miteinander vermischt, beispielsweise durch Rühren. Nach dem Ende der Reaktion wird das Reaktionsgemisch abgekühlt und der Feststoff abgesaugt, mit Wasser nachgewaschen und getrocknet. Vorteilhaft für das Verfahren ist das Arbeiten mit einem 5- bis 20fachen molaren Überschuß an Ammoniak bezogen auf die 4,5-Dichlorverbindung sowie die Verwendung einer 5- bis 30%igen (Gew.-%) wäßrigen Ammoniak-Lösung. Bei der Umsetzung mit Ammoniak liegt die Phenol-4-sulfonsäure in Form ihres Ammoniumsalzes vor. Unter dem allgemeinen Ausdruck »Phenol-4-sulfonsäure« ist also auch ihr Ammoniumsalz zu verstehen.

Die Umsetzung wird bei einer Temperatur von 20 bis 200°C, vorzugsweise bei mindestens 80°C, zweckmäßig bei 100 bis 150°C, insbesondere bei 110 bis 130°C durchgeführt.

Zweckmäßig wird das Verfahren in Gegenwart von 1 bis 100 Molprozent Phenol-4-sulfonsäure, bezogen auf die 4,5-Dichlorverbindung, vorzugsweise bei mindestens 10 Molprozent, vorteilhaft bei 20 bis 70 Molprozent, insbesondere bei 30 bis 50 Molprozent durchgeführt.

Die Umsetzung wird bei erhöhtem Druck (1 bar bis 50 bar) vorzugsweise bei 3 bis 10 bar, insbesondere bei dem Druck durchgeführt, der sich im geschlossenen Reaktionsgefäß bei der Erreichung der Reaktionstemperatur einstellt. Es ist auch möglich, während der Umsetzung im geschlossenen Gefäß durch Einpressen von Ammoniak den Druck zu erhöhen. Hierbei löst sich ein Teil des eingepreßten Ammoniaks in der wäßrigen Ammoniaklösung.

Das erfindungsgemäße Verfahren ist sehr umweltfreundlich, weil die bei der Abtrennung des Reaktionsproduktes erhaltene Mutterlauge gegebenenfalls nach Zusatz von Ammoniak, wäßriger Ammoniaklösung oder Phenol-4-sulfonsäure zur erneuten Umsetzung mit weiterem 4,5-Dichlorderivat verwendet werden kann. Die Mutterlauge wird also vollständig oder z. T. wiederverwendet.

Die folgenden Beispiele zeigen die Einfachheit und Zuverlässigkeit des erfindungsgemäßen Verfahrens.

Beispiel 1

In einem Druckgefäß, das mit einem Heizmantel versehen ist und ein Volumen von 300 Volumenteilen besitzt, werden 85 Volumenteile 15%igen (Gew.-%) wäßriger Ammoniak, 12,05 Gewichtsteile 4,5-Dichlor-1-phenyl-pyridazon-(6) und 6,2 Gewichtsteile einer 70%igen wäßrigen Lösung von Phenol-4-sulfonsäure eingefüllt (Volumenteile verhalten sich zu Gewichtsteilen wie Liter zu Kilogramm). Die Reaktionsmischung wird nun unter Rühren auf 130°C erhitzt und 4 Stunden bei dieser Temperatur gehalten. Dabei bildet sich ein Druck von 6 bar. Danach läßt man auf Raumtemperatur (20°C) abkühlen und saugt die Mutterlauge vollständig von der Reaktionsmischung ab. Man trennt danach die Mutterlauge ab, wäscht das feste Reaktionsprodukt mit Wasser nach und trocknet im Vakuum bei 100°C. Man erhält 9,8 Gewichtsteile (89% d. Th.) 4-Amino-5-chlor-1-phenylpyridazon-(6) vom Schmelzpunkt 205 bis 206°C (Gehalt an 5-Amino-4-chlor-1-phenyl-pyridazon-(6) gaschromatographisch bestimmt: 1%, Gehalt an 4-Aminoderivat 99%).

Beispiel 2

Die in Beispiel 1 abgetrennte Mutterlauge, 12,05 Gewichtsteile 4,5-Dichlor-1-phenyl-pyridazon-(6) und 15 Volumenteile 15%iger wäßriger Ammoniak werden gemischt und wie im Beispiel 1 beschrieben 6 Stunden auf 130°C gehalten. Nach der Aufarbeitung erhält man 10,1 Gewichtsteile (91% d. Th.) 4-Amino-5-chlor-1-phenyl-pyridazon-(6) vom Schmelzpunkt 202 bis 204°C (Gehalt an 5-Amino-4-chlor-1-phenyl-pyridazon-(6) gaschromatographisch bestimmt: 2,3%, Gehalt an 4-Aminoderivat: 96%).

**Patentansprüche**

1. Verfahren zur Herstellung von weitgehend reinem 4-Amino-5-chlor-1-phenyl-pyridazon-(6) durch Umsetzung von 4,5-Dichlor-1-phenyl-pyridazon-(6) mit wäßrigem Ammoniak unter Druck und erhöhter Temperatur, dadurch gekennzeichnet, daß man die Umsetzung mit einer wäßrigen Lösung von Ammoniak bei einer Temperatur von 20 bis 200°C und einem Druck von 1 bis 50 bar in Gegenwart von Phenol-4-sulfonsäure durchführt.

2. Verfahren nach Anspruch 1, dadurch gekennzeichnet, daß man das 4-Amino-5-chlor-1-phenyl-pyridazon-(6) nach der Umsetzung von der Reaktionsmischung abtrennt und die zurückbleibende Mutterlauge, gegebenenfalls nach Zusatz von Ammoniak, wäßriger Ammoniaklösung oder Phenol-4-sulfonsäure, zur erneuten Umsetzung mit weiterem 4,5-Dichlor-1-phenyl-pyridazon-(6) verwendet.

**Claims**

1. A process for the manufacture of substantially pure 4-amino-5-chloro-1-phenylpyridaz-6-one by reaction of 4,5-dichloro-1-phenylpyridaz-6-one with aqueous ammonia at superatmospheric pressure and elevated temperature, characterized in that the reaction is carried out with an aqueous solution of ammonia, at a temperature of from 20° to 200°C and a pressure of from 1 to 50 bars, in the presence of phenol-4-sulfonic acid.

2. A process as claimed in claim 1, characterized in that the 4-amino-5-chloro-1-phenylpyridaz-6-one is separated from the reaktion mixture after the reaction, and the remaining mother liquor is, if desired after the addition of ammonia, aqueous ammonia solution or phenol-4-sulfonic acid, employed again for reaction with further 4,5-dichloro-1-phenylpyridaz-6-one.

**Revendications**

1. Procédé de préparation de 4-amino-5-chloro-1-phényl-pyridazone-(6) de pureté élevée par réaction de 4,5-dichloro-1-phényl-pyridazone-(6) sous pression et à température accrue avec l'ammoniaque, caractérisé en ce que l'on effectue la réaction à une température de 20 à 200°C et sous une pression de 1 à 50 bars dans une solution d'ammoniaque en présence d'acide phénol-4-sulfonique.

2. Procédé suivant la revendication 1, caractérisé en ce que, la réaction étant terminée, la 4-amino-5-chloro-1-phényl-pyridazone-(6) est séparée du mélange réactionnel et la lessive mère est réutilisée pour une nouvelle réaction avec la 2,5-dichloro-1-phényl-pyridazone-(6), le cas échéant après addition d'ammoniac, d'ammoniaque ou d'acide phénol-4-sulfonique.